(19) Europäisches Patentamt
European Patent Office
Office européen des brevets

(11) Publication number: **0 054 432**
**B1**

(12) **EUROPEAN PATENT SPECIFICATION**

(45) Date of publication of patent specification: **31.01.90**

(21) Application number: **81305874.0**

(22) Date of filing: **14.12.81**

(51) Int. Cl.[5]: **C 07 D 409/04,** A 61 K 31/425
// C07H19/04

(54) **Use of 2-Beta-D-ribofuranosylthiazole-4-carboxamides for the manufacture of medicaments inhibiting malignant tumors.**

(30) Priority: **15.12.80 US 216197**
**24.11.81 US 324455**

(43) Date of publication of application:
**23.06.82 Bulletin 82/25**

(45) Publication of the grant of the patent:
**31.01.90 Bulletin 90/05**

(84) Designated Contracting States:
**AT BE CH DE FR GB IT LI LU NL SE**

(56) References cited:
**FR-A-2 279 410**
**US-A-3 998 999**

**Europ. J. Cancer 13 (1977), 821**

**JOURNAL OF MEDICINAL CHEMISTRY, vol. 20, no. 2, February 1977, pages 256-262, P.C. SRIVASTAVA et al.: "Synthesis and antiviral activity of certain thiazole G-nucleosides"**

**The file contains technical information submitted after the application was filed and not included in this specification**

(73) Proprietor: **VIRATEK, INC.**
**222 North Vincent**
**Covina California 91722 (US)**

(72) Inventor: **Robins, Roland Kenith**
**406 Sherwood**
**Provo Utah 84601 (US)**

(74) Representative: **Jones, Michael Raymond et al**
**HASELTINE LAKE & CO. Hazlitt House**
**28 Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 054 432 B1

## Description

This invention relates to the use of the compound 2-β-D-ribofuranosylthiazole-4-carboxamide and related compounds (such as esters) for the manufacture of medicaments for inhibiting malignant tumors, and processes for producing certain compounds.

Complete control of malignant tumors in man and other warm blooded animals still remains an unrealized goal. Within the last few decades, understanding of malignancy has made significant progress; however, conquest of the malignant disease state has not been realized.

Conventional therapy of both humans and other valuable animal species inflicted with malignant tumors presently includes surgical excision of the tumor, local radiation therapy of the afflicted patient, and chemotherapy by administration of a chemotherapeutic agent to the patient. The death of a significant number of patients inflicted with malignant tumors is attributable not to the primary tumor but instead to metastastis of the primary tumor to secondary sites in the host. If a primary tumor is detected early, it can normally be eliminated by surgery, radiation or chemotherapy or combinations of these. The metastatic colonies of these primary tumors, however, are considerably harder to detect and eliminate, and the unsuccessful management of them remains a serious medical problem.

Tumors are normally classified either as benign or malignant. The malignant tumor is distinguished from the benign by its ability to invade both surrounding tissue and to colonize distant sites via metastisis. Certain organs are more prone to metastisis than others, and included in this group are the lung, the brain, the liver, the ovaries and the adrenal glands. It has further been suggested that both surgery and radiation of a primary tumor in certain instances actually promotes metastisis.

In view of the inability of current cancer therapy to control successfully the malignant tumor and its metastisis, it is evident that there exists a need for additional chemotherapeutic agents.

In a paper entitled Synthesis and Antiviral Activity of Certain Thiazole C-Nucleosides, *J. Med. Chem.* 1977, *Volume 20,* No. 2,256, Professor R. K. Robins et al disclosed the synthesis of, and certain preliminary in vitro antiviral activity of, the compounds 2-β-D-ribofuranosylthiazole-4-carboxamide (sometimes hereinafter referred to as the "parent compound) and 2-(2,3,5-tri-O-acetyl-β-D-ribofuranosyl)thiazole-4-carboxamide. These two compounds were used in an in vitro test system utilizing three viruses, namely type 1 herpes simplex virus, type 3 parainfluenza virus and type 3 rhinovirus. The in vitro activity of the compound 2-β-D-ribofuranosylthiazole-4-carboxamide against these three viruses was only moderate. With the compound 2-(2,3,5-tri-O-acetyl-β-D-ribofuranosyl)thiazole-4-carboxamide, only moderate activity was seen with type 1 herpes simplex virus whereas with the type 3 parainfluenza virus and the type 13 rhinovirus the activity was negative. While, as indicated, certain marginal in vitro antiviral activity was observed, then, quite to the contrary, in vivo antiviral testing for both 2-β-D-ribofuranosylthiazole-4-carboxamide and 2-(2,3,5-tri-O-acetyl-β-D-ribofuranosylthiazole, as judged by the number of test animal deaths, was negative. In the in vivo tests, the number of deaths for the test animals for both the 2-β-D-ribofuranosylthiazole-4-carboxamide and 2-(2,3,5-tri-O-acetyl-β-D-ribofuranosylthiazole-4-carboxamide equalled or exceeded the number of deaths of the placebo control animals indicating that both of the compounds 2-β-D-ribofuranosylthiazole-4-carboxamide and 2-(2,3,5,O-acetyl-β-D-ribofuranosylthiazole-4-carboxamide demonstrated no useful in vivo antiviral activity.

With regard to the above noted in vitro antiviral testing for both 2-β-D-ribofuranosylthiazole-4-carboxamide, and 2-(2,3,5-tri-O-acetyl-β-D-ribofuranosyl)thiazole-4-carboxamide, these compounds were tested against viruses for which the known antiviral compound Ribavirin (registered Trade Mark) is known to have positive antiviral activity. Rivavirin and related compounds in the aforementioned paper inhibit guanine nucleotide bisynthesis as evidenced by Ehrlichascites tumor cell suspensions; this is a model which is not considered as a suitable model for testing antitumor activity. In view of the preliminary marginal in vitro activity of 2-β-D-ribofuranosylthiazole-4-carboxamide against these test viruses, it was expected that the spectrum of activity of 2-β-D-ribofuranosylthiazole-4-carboxamide would be similar to the spectrum of activity of the compound Ribavirin. Ribavirin is known to be an active in vitro antiviral agent and in vivo antiviral agent and is further known to exhibit no significant antitumor activity. Additionally, certain derivatives of Ribavirin, such as its 5'monophosphate, are also known to be inactive as antitumor compounds. It was reasonable to expect, in comparing the preliminary in vitro antiviral activity of 2-β-D-ribofuranosylthiazole-4-carboxamide with that of Ribavirin, that 2-β-D-ribofuranosylthiazole-4-carboxamide would exhibit positive in vivo antiviral activity and negative antitumor activity similar to Ribavirin. Totally contrary to expectations, the compound 2-β-D-ribofuranosylthiazole-4-carboxamide possessed no useful in vivo antiviral activity and, quite unexpectedly, has demonstrated positive antitumor activity.

In support of the statement that Ribavirin, while a known antiviral compound, has no antitumor activity, reference is made to the article "The Effects of 1-β-D-ribofuranosyl-1,2,4-Triazole-3-Carboxamide (Ribavirin) on the Transplanted Tumors of Animals" in Europ. J. Cancer, Vol. 13, pp 821—830. The conclusion of the article, on page 829, indicates, in the last sentence, that Ribavirin probably has no value in antitumor therapy, and may even have deleterious effects.

US—A—3998999 and FR—A—2279470 merely disclose C-glycosyl-heterocycles, in fact 4-hydroxypyrazole-5-carboxamides, which are not structurally similar to the thiazole-4-carboxamides of the present invention as Ribavirin. It has already been indicated above why Ribavirin is thought not to have any antitumour characteristics.

Insofar as the compounds disclosed in US—A—3998999 and FR—A—2279470 have some pharmaceutical activity, those compounds necessarily contain a nitrogen and a sulphur atom where the sulphur atom is interrupted by a carbon atom between the nitrogen and the sulphur. Ribavirin also does not have a 4-hydroxy group but has 3 nitrogen atoms in the heterocyclic ring, with one nitrogen being spaced from the other nitrogen by a carbon, and the other being next to one another. The dissimilarity between the compounds disclosed in FR—A—2279470 and US—A—3998999 would not lead an artisan to attribute to the compounds of the present invention the pharmaceutical properties which they in fact possess.

Thus, a basis of the present invention is the discovery that the compound 2-β-D-ribofuranosylthiazole-4-carboxamide and its esters, including 2-(2,3,5-tri-O-acetyl-β-D-ribofuranosyl)thiazole-4-carboxamide and 2-(5-O-phosphoryl-β-D-ribofuranosyl)thiazole-4-carboxamide exhibit antitumor activities of such significance as to be useful as antitumor agents in vivo.

According to one aspect of the present invention, there is provided the use for the manufacture of a medicament for inhibiting a malignant tumor in a warm blooded animal of a compound having the following general formula (I):

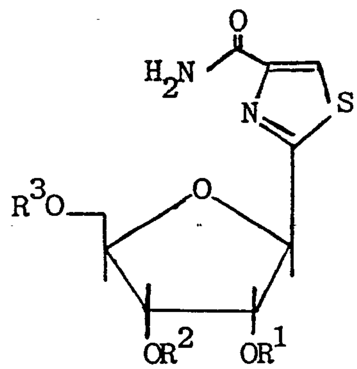

I

wherein each of $R^1$ and $R^2$, which are the same or different, is a hydrogen atom or a $C_{1-18}$ carboxylic acyl radical, and $R^3$ is a hydrogen atom, a $C_{1-18}$ carboxylic acyl radical or

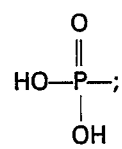

or a physiologically acceptable salt thereof.

The term "acyl" used herein means "carboxylic acyl".

Preferably, when any of $R^1$, $R^2$ and $R^3$ is an acyl radical, that radical is a $C_{1-18}$ acyl radical. Conveniently at least one of the radicals $R^1$, $R^2$ and $R^3$ is a benzoate ester radical.

A preferred compound of formula (I), for the use according to the invention, is 2-(5-O-phosphoryl-β-D-ribofuranosyl)thiazole-4-carboxamide and physiologically acceptable salts thereof.

A further aspect of the present invention provides the use for the manufacture of a medicament for inhibiting a malignant tumor in a warm blooded animal of a composition containing, in addition to a physiologically acceptable carrier and as an active ingredient, an effective amount of a compound of formula (I), or a physiologically acceptable salt thereof.

The active compound in the composition may be 2-β-D-ribofuranosylthiazole-4-carboxamide; or an acyl derivative thereof, for instance where the acyl ester is an acetate ester or benzoate ester; or a phosphoryl ester thereof.

The present invention also provides a process for producing a compound having the following general formula:

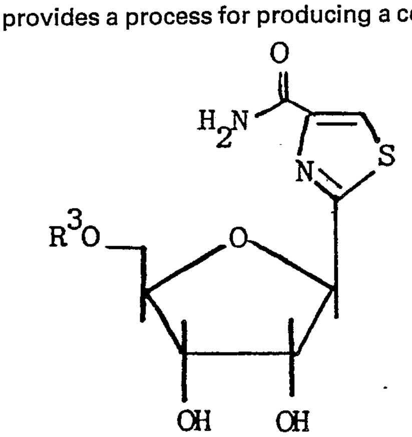

wherein $R^3$ is a $C_{1-18}$ acyl radical or

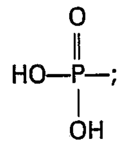

which process comprises reacting the compound 2-β-D-ribofuranosylthiazole-4-carboxamide in the presence of a base with a compound selected from an acyl anhydride, an acyl chloride, a phosphoric acid anhydride or a phosphoric acid chloride.

The present invention further provides a process for producing 2-(5-O-phosphoryl-β-D-ribofuranosyl)-thiazole-4-carboxamide, which process comprises reacting the compound 2-β-D-ribofuranosylthiazole-4-carboxamide in the presence of a base with phosphoryl chloride in a polar solvent; and treating the reaction mixture with water and isolating the desired product.

Conveniently the desired product is isolated by desalting the reaction mixture with activated charcoal and eluting the product out of the charcoal.

The compound 2-β-D-ribofuranosylthiazole-4-carboxamide has been shown to exhibit significant antitumor activity in vivo. The present invention relates to the use of this compound and certain related derivatives in the preparation of a medicament for treating malignant tumors in warm blooded animals. The antitumor properties of 2-β-D-ribofuranosylthiazole-4-carboxamide and its related esters may be utilized by administering to a warm blooded animal an effective amount of a pharmaceutical composition containing as the active compound at least 0.1 percent by weight, based on the total weight of the composition, of a compound of the following general formula (I)

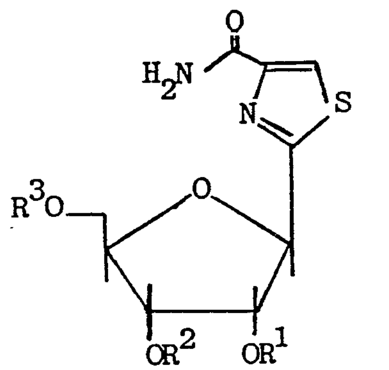

I

wherein $R^1$, $R^2$ and $R^3$ are as defined above, or a physiologically acceptable salt thereof.

Specifically noted for $R^1$, $R^2$ and $R^3$ as preferred acyl groups are acetyl, propionyl, butyl, isobutyl and benzoyl. Specifically noted as acceptable salts are the alkali metal and ammonium or substituted ammonium salts such as sodium, potassium and ammonium salts.

Preferably, when $R^1$ and $R^2$ are H, $R^3$ is H, $C_{1-18}$ acyl or

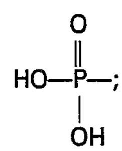

and, when $R^1$ and $R^2$ are $C_{1-18}$ acyl, $R^3$ is $C_{1-18}$ acyl.

For use in the pharmaceutical composition of the present invention, a pharmaceutical carrier would be utilized such that, preferably, the pharmaceutical carrier would be chosen to allow administration of a suitable concentration of the active compounds of the present invention as solutions or suspensions by injection into an afflicted warm blooded animal. Depending on the host harbouring the malignant tumor, the type of tumor, and the tumor site, administration by injection would be intraveneously, intramuscularly, intracerebrally, subcutaneously, or intraperitoneally.

Alternatively, the composition of the present invention might suitably be formulated in appropriate pharmaceutical carriers allowing for administration by other routes such as oral administration, ophthalmic administration, topical administration or administration by suppository.

The parent compound of the present invention, the compound 2-β-D-ribofuranosylthiazole-4-carboxamide (which is also referred as Compound 1, as it is the product of Example 1), is preferredly prepared as described in the following Example 1. An alternative synthesis of this compound appears in *J. Org. Chem.* Vol. 41, No. 26, 1976, 4074.

Certain esters of 2-β-D-ribofuranosylthiazole-4-carboxamide, Compound 1, such as 2-(2,3,5-tri-O-acetyl-β-D-ribofuranosyl)thiazole-4-carboxamide, Compound 2 or 2-(5-O-phosphoryl-β-D-ribofuranosyl)-thiazole-4-carboxamide, Compound 3, are prepared as described in Examples 2 and 3 respectively. Additionally, other esters, such as the monoester 2-(5-O-acetyl-β-D-ribofuranosyl)thiazole-4-carboxamide, Compound 4, can be prepared as in the synthesis described in Example 4. For other preferred carboxylic esters of the present invention, substitution of acetic anhydride with a suitable anhydride such as propionic anhydride, butyric anhydride or benzoic anhydride is made. Alternatively, the appropriate acid chloride could be substituted for the acid anhydride.

The esters of Compound 1 can assist in delivery of the compound in an afflicted host. Such esters of the compound can be formed by reacting one or more of the hydroxyl groups of the sugar moiety of Compound 1 with suitable reversible blocking groups which could be cleaved in vivo from the parent Compound 1 by certain in situ chemical or enzymatic reactions.

For reaction with the hydroxyl groups, esters such as, but not necessarily limited to, acyl and phosphoryl esters can be considered. The acyl groups can be those of acids selected from straight chain, branch chain, substituted, unsaturated, saturated or aromatic acids such as, but not necessarily limited to, acetic, trifluoroacetic, propionic, *n*-butyric, isobutyric, valeric, caproic, pelargonic, enanthic, capyrlic, lactic, arcylic, propargylic, palmitic, benzoic, phthalic, salicylic, cinnamic and naphthoic acids. If a phosphoryl group is chosen, the phosphoryl ester could be as a free acid or as a salt form. Acceptable salts of the phosphate moiety of the phosphoryl ester can be selected from, but not necessarily limited to, the group of alkali metal and alkaline earth metal salts, e.g. sodium, potassium, calcium, magnesium, lithium, ammonium and substituted ammonium trialkylammonium, dialkylammonium, alkylammonium, e.g. triethylammonium, trimethylammonium, diethylammonium, octylammonium, cetyltrimethylammonium and octylpyridinium.

As preferred agents in the form of esters of the present invention, Compounds 2, 3 and 4 are mentioned. In addition to these, other tri-O-acyl esters such as the 2′,3′,5′-tri-O-benzoyl may be mentioned. Additionally, other mono-esters such as the 5′-O-benzoyl can be considered. Generally, for carboxylic esters the preferred esters would include $C_{1-18}$ acyls, a more preferred group being $C_{1-8}$ acyls. Preferredly, when phosphoryl esters are utilized, the phosphate groups would be formed as a salt, preferably as a sodium salt or other alkali metal salt or ammonium.

Ester forms of Compound 1, as is shown in the examples herein, are useful in delivering the compound to the affected site in an affected host. As is shown in the examples, the tri-acetyl ester, Compound 2, is indicated as being an effective antitumor agent when injected intraperitoneally into an affected host. The described tri-acetyl compound and any other acyl ester of Compound 1 would be expected to be hydrolyzed to Compound 1 in certain biological fluids such as the acid environment of the stomach or an environment which includes an appropriate enzyme capable of in vivo enzymatic cleavage of the ester to Compound 1. Without wishing to be bound by theory, if the phosphoryl ester of Compound 1, such as the 5′-phosphate, were used, other enzymes present in vivo might also be expected appropriately to cleave enzymatically the phosphate to yield an in situ delivery of Compound 1. Compound 3, the phosphoryl ester of Compound 1, as is shown in the examples, is indicated as being an effective antitumor agent when injected in an effected host. At this time it is not known whether its activity is expressed as the 5′-phosphate or whether it is enzymatically cleaved to Compound 1. Further, it is possible that Compound 1 might be promoted in situ by other enzymatic reactions to Compound 3. In any event, both Compound 1 and Compound 3 are indicated as being effective in vivo antitumor agents as is indicated by the examples.

In performing the present invention, Compound 1, or a selected ester form thereof, is appropriately mixed with a suitable pharmaceutical carrier which may be as simple as sterilized water or could be a complex carrier having appropriate agents suitably to mimic certain biological environments, i.e. a pH and salt adjusted solution suitable for intraveneous, intramuscular or other injections.

In selecting a suitable pharmaceutical carrier, consideration should be made to the type of tumor, the site of the tumor and the health and age of the host. Additionally, if an ester form of Compound 1 is used, consideration of the chemical reactivity of the ester should also take place. Thus, carboxylic acyl ester would preferredly be suspended or solubilized in an appropriate non-acidic medium. A phosphoryl ester might be appropriately used in the presence of a suitable buffer or as a salt as discussed above.

Preferredly, Compound 1 or any of the other agents or compounds of the present invention, is mixed with an appropriate pharmaceutical carrier such that Compound 1 or a derivative thereof would be suitably soluble in the carrier. Alternatively, however, suspensions, emulsions and other formulations of the compounds of the present invention could be used where indicated. The pharmaceutical carrier, in addition to having solubilizing or suspending agent therein, might also include suitable dilutants, buffers, surface active agents and other similar agents as are typically used in pharmaceutical carriers. The total composition of the pharmaceutical carrier would, however, be chosen to be compatible with the site of delivery, the concentration of the active ingredient and other parameters as are standard in pharmaceutical industry.

Compound 1, or the other agents or compounds of the invention, would be suitably mixed with the pharmaceutical carrier such that it would be present in a concentration of at least 0.1 percent by weight of the total composition. Preferredly, it would be present in the pharmaceutical carrier at a concentration of from 10% to 90% by weight of the total composition.

Effective amounts of Compound 1, or the other compounds of the present invention, typically range from 2.5 milligrams per kilogram (mg/kg) of the total body weight of the treated warm blooded animal to

200 mg/kg per day. Preferably, the range is from 12.5 mg/kg to 100 mg/kg, per day. An even more preferred range would be from 15 mg/kg to 50 mg/kg, per day. As with other factors noted above, the amount of compound utilized in treating an afflicted animal would take into account parameters such as the type of tumor, the tumor site, the form of administering the compound and the physical size and condition of the host. In any event, the actual amount should be sufficient to provide a chemotherapeutically effective amount of the agent to the host in a convenient volume, which will be readily within the ability of those skilled in the art to determine given the disclosure herein.

In at least one study, Compound 1 of the invention has been injected at dosages up to 2000 mg/kg into tumor-bearing animals and no deaths of the animals were attributed to the toxicity of Compound 1 on the toxicity day of the test. In a host which has been diagnosed as being terminally ill with a malignant tumor excessive amounts beyond any toxicity range might be indicated if there is any probability of cure of the terminally ill host is commonly practised in current cancer chemotherapy.

As in the examples used for illustrative purposes below, a tumor bearing host was treated once daily with the indicated test compound. Depending upon the clinical situation, the daily dose of Compound 1 or any of the other compounds of the present invention, might be similarly given; however, the daily dose could also be broken up into sub-unit doses which, in their totality, equal the daily dose. Thus, for example, at a 50 mg/kg dose level the patient might be appropriately treated four times a day with doses of 12.5 mg/kg.

A composition used for inhibiting malignant tumors in warm blooded animals might be suitably prepared by adding Compound 1 or any of the other compounds of the invention, to a pharmaceutically compatible solvent followed by sterilization and packaging in appropriate sealable vials at a known concentration. Appropriate doses of the compound are then withdrawn from the vial and administered by injection to the host.

Example 1

2-β-D-Ribofuranosylthiazole-4-Carboxamide, COMPOUND 1 (known from J. Med. Chem. *20*, 2 (1977), 256)

Ethyl 2-(2,3,5-tri-o-benzoyl-β-D-ribofuranosyl)thiazole-4-carboxamide was utilized as prepared in Srivastova et al, J. Med. Chem, 1977, Volume 20, No. 2, 256. A concentrated solution of ethyl 2-(2,3,5-tri-O-benzyl-β-D-ribofuranosyl)thiazole-4-carboxamide (5.0 g, 8.31 mmole) in methanol (15 ml) was stirred with methanolic ammonia (saturated at 0°C, 100 ml) in a pressure bottle at room temperature for 2 days. The solvent was evaporated and the residue was chromatographed through a column (2.5 × 35 cm) of silica gel (100 g) packed in ethyl acetate. Elution of the column with a solvent system (ethyl acetate-1-propanol-water, 4:1:2, v/v; top layer) removed the fast-moving methyl benzoate and benzamide. The slower moving, major, UV and sugar-positive fractions were collected and the solvent was evaporated in vacuo. The residue (syrup), thus obtained, was readily crystallized from ethanol-ethyl acetate to provide 1.6 g (74%) of pure product, compound 1: mp 144—145°C; $[\alpha]^{25}$p-14.3°C (c 1, DMF); UV$\lambda_{max}$ pH1 237 nm (8640); UV$\lambda_{max}$ pH11 238 nm (8100); $^1$HNMR($Me_2SO$-$d_6$)δ 7.5—7.8 [S(br), 2, $CONH_2$)$^1$HNMR ($Me_2SO$-$d_6$-$D_2O$)δ4.99 (d, 1, J—5Hz, $H_{1'}$), 8.25 (s, 1, $H_5$). Anal ($C_9H_{12}N_2O_5S$) C, H, N, S.

Example 2

2-(2,3,5-Tri-O-Acetyl-β-D-Ribofuranosyl)thiazole-4-Carboxamide, COMPOUND 2 (known from J. Med. Chem. *20,* 2 (1977), 256

Acetic anhydride (2.0 ml) was added to an ice-cold solution of compound 1 (1.04 g, 4 mmol) in anhydrous pyridine (16 ml) and the reaction solution was stirred at room temperature for 17 h. The solvent was evaporated in vacuo, the residue was dissolved in ethyl acetate, and the solution was washed with water and dried ($MgSO_4$). The ethyl acetate portion was evaporated in vacuo and the residue thus obtained was crystalized from water to provide 1.4 g (90%) of compound 2 as white needles; mp 103°C; $^1$HNMR ($CDCl_3$) 2.1 (3, s, 9, tri-O-acetyl) 6.2 and 7.15 [pair of s(br), 2, $CONH_2$); 8.2 (s, 1, $H_5$). Anal. ($C_{15}H_{18}N_2O_8S$) C, H, N, S.

Example 3

2-(5-O-Phosphoryl-β-D-Ribofuranosyl)thiazole-4-Carboxamide, (2-β-D-Ribofuranosyl)thiazole-4-Carboxamide 5′-Phosphate), COMPOUND 3

Water (151 mg, 8.4 mmol) was added carefully to a solution (maintained at 0°C with stirring) of freshly distilled phosphoryl chloride (2.0 g, 13.2 mmol), pyridine (1.21 g, 14.4 mmol) and acetonitrile (2.3 g, 56.7 mmol). 2-β-D-Ribofuranosyl)thiazole-4-carboxamide, compound 1, (dried over $P_2O_5$ and powdered, 800 mg, 3.0 mmol) was added to the solution and the reaction mixture was stirred continuously for 4 hrs at 0°C. The reaction mixture was poured into ice water (ca. 50 ml) and the pH was adjusted to 2.0 with 2N sodium hydroxide. The solution was applied to a column of activated charcoal (20 g), and the column was washed thoroughly with water until the eluate was salt-free. The column was eluted with a solution ethanol-water-concentrated ammonium hydroxide (10:10:1) and the fractions (25 ml each) were collected. The fractions containing pure (tlc, silica gel, acetonitrile-0.1 N ammonium chloride (7:3)) nucleotide, compound 3, were collected and evaporated to dryness under vacuum. The anhydrous residue was dissolved in water and passed through a column of Dowex 50—X8 (20—50 mesh, $H^+$ form, 15 ml). The column was washed with water and the fraction containing the nucleotide was collected. The solution was concentrated to a small volume (5 ml) and passed through a column of Dowex 50W—X8 (20—50 mesh, $Na^+$ form, 15 ml). The column was washed with water. The nucleotide containing fraction was lyophilized. The

residue was triturated with ethanol, collected by filtration and dried ($P_2O_5$), to provide 560 mg (47%) of compound 3 as monosodium dihydrate in the crystalline form.

| | | | | | |
|---|---|---|---|---|---|
| Anal. calcd. for $C_9H_{12}N_2O_8PSNa \cdot 2H_2O$: | C, 27.13; | H, 4.04; | N, 7.04; | P, 7.78; | S, 80.5. |
| Found: | C, 27.42; | H, 3.87; | N, 7.07; | P, 80.3; | S, 8.41. |

Example 4

2-(5-O-Acetyl-β-D-Ribofuranosyl)thiazole-4-carboxamide COMPOUND 4

A solution of 2-(2,3-O-isopropylidene-β-D-ribofuranosyl)thiazole-4-carboxamide (1.5 g, 5 mmol) (prepared as per Fuertes, et al, J. Org. Chem., Volume 41, No. 26, 1976, 4074) in anhydrous pyridine (20 ml) was chilled in an ice-water bath and acetic anhydride (2.5 ml) was slowly added with stirring. The reaction solution was allowed to warm to room temperature and stirring was continued for 15 h. The solvent was evaporated in vacuo and the residue was dissolved in ethyl acetate and washed with water. The ethyl acetate portion was evaporated in vacuo and the residue was dissolved in 80% acetic acid (25 ml). The solution was heated on a steam bath for 30 mins, and the solvent evaporated in vacuo. The residue was dissolved in ethyl acetate, washed once with water and dried over $MgSO_4$. The ethyl acetate portion was evaporated and the crude product was passed through a column of silica gel (100 g, packed in chloroform) and eluted with 20% (V/V) ethyl acetate in chloroform. The nucleoside bearing fractions were pooled and evaporated to yield 1.05 g (70%) of compound 4. ($C_{11}H_{14}N_2O_6S$).

As illustrative examples of the use of compound 1 and other illustrative compounds of the invention, examples 5 through 12, below, are given. In these examples, the efficacy of the compounds is demonstrated using the standard tests against certain malignant tumors. The tests utilized in these illustrative examples were conducted by the Developmental Therapeutics Program, Division of Cancer Treatment, National Cancer Institute. The tests were supervised by this agency, utilizing their standard protocols and procedures. All tests conformed to these protocols and all tests were evaluated under the criteria defined by these protocols. The following representative examples illustrate confirmed activity of the illustrative compounds of the invention against screening tumor systems of the National Cancer Institute.

For purposes of the following examples, the abbreviation IP stands for intraperitoneal and IV stands for intravenous. The mean and median survival times are calculated in instruction 14 (revised 6/78) of the Screening Data Summary, Developmental Therapeutics Program, Division of Cancer Treatment, National Cancer Institute. The contents of this Screening Data Summary including appropriate revisions are herein incorporated by reference.

In the illustrated examples below, the vehicle used as carrier for the drug was injected (minus any drug therein) into the control animals at the same level of use of the vehicle in the drug treated animals in order to eliminate any vehicle effect of the tests.

Example 5

As an indicator of reproducible activity, compound 1 of the invention was screened against L—1210 lymphoid leukemia in vivo using $CD_2P_1$ male mice as the testing species. The parameter of efficacy chosen was based upon the medium survival time of animals treated with the drug vs. appropriate control group animals. Both drug treated animals and control group animals were inoculated IP with $10^5$ seed cells of L—1210 lymphoid leukemia in Ascitic fluid.

One day after tumor inoculation, the drug group of animals was placed on a regimen of treatment of compound 1 at the dose levels as noted below, table 1. The drug treated group of animals was inoculated once daily for five days at the doses noted by IP injection of the test compound appropriately diluted with water.

Day six was chosen as an indication of drug toxicity. In this example, all drug animals survived through day six. Death of drug treated animals after day six was, therefore, attributed to tumor deaths and not drug toxicity.

The median death day of the control group was day 8.5. As noted in table 1 below, the median death day of the drug treated group was longer at all levels of drug tested and was significantly longer at greater than 50 mg/Kg (amount of drug/weight of test animal). The results shown in table 1 below indicate that in this multiple dose assay, the drug showed positive activity. A percent of drug treated animals/control animals greater than 125% is taken as positive drug activity.

TABLE 1

| Drug Dose mg/Kg | Treated Group Survival Time | Control Group Survival Time | Percent Treated Animals/ Control Animals |
|---|---|---|---|
| 200 | 14.3 | 8.5 | 168% |
| 100 | 12.7 | | 149% |
| 50 | 11.0 | | 129% |
| 25 | 10.2 | | 120% |
| 12.5 | 9.5 | | 111% |

Example 6

Compound 2, 2-(2,3,5-tri-O-acetyl-β-D-ribofuranosyl)thiazole-4-carboxamide was screened in a manner similar to that shown in Example 5, however, the tumor system used as the test tumor was P388 lymphocytic leukemia. $10^6$ seed cells were used to initiate the tumor in both the control group and the drug treated group of animals. The same strain of mice was used except female mice were substituted for males. Test results were based on mean survival time and are expressed as T/C percentages (treated animals/control animals) as per Example 5.

In the drug treated animals, treatment was initiated one day after tumor inoculation and the drug was given at the dose levels noted below in table 2. Drug treatment was for nine days and drug toxicity, as in Example 5, was measured on day six. At the 100 mg/Kg level, one animal did not survive the toxicity cut-off date.

Average day of death for the control group was 10.2 days whereas at the lowest level of drug treatment the treated animals survived for more than 15 days. As with Example 5, 125% increase in longevity of treated animals over control animals was taken as indicative of positive drug response.

TABLE 2

| Drug Dose mg/Kg | Treated Group Survival Time | Control Group Survival Time | Percent Treated Animals/ Control Animals |
|---|---|---|---|
| 200 | 18.3 | 10.2 | 179% |
| 100 | 18.0 | | 176% |
| 50 | 15.3 | | 150% |

Compound 1 is also indicated as being active against P388 lymphocytic leukemia as per Examples 6a, b, and c, and 2-(2,3,5-tri-O-acetyl-β-D-ribofuranosyl)thiazole-4-carboxamide, compound 2, is indicated also as being active against P388 lymphocytic leukemia as per Example 7. In both of these examples, the compound successfully passed the DN 2 (Decision Network) criteria of the National Cancer Institute Testing. For examples 7 and 8, $CD_2F_1$ female mice were used and challenged with P388 lymphocytic leukemia tumors. The median survival time of the drug treated animals was compared to appropriate control animals and based on this criteria both of the compounds tested were considered as active antitumor agents. The test period was for thirty days in both Examples 7 and 8.

For Examples 7 and 8, as well as Examples 9 and 10 below, any animal of the drug treated group which survived beyond the end of the testing period was then evaluated and placed in one of three groups. The first group was designated as cured, meaning the animal was successfully cured of the tumor. The second group designation was no-takes, meaning survival of the animal was considered to be due to failure of the tumor implant. The remaining group was designated as tumor survivors meaning the animals lived beyond the test cut-off date but could not be classified as either cured or no-takes.

For both Examples 7 and 8, thirty animals were used as the control group and six animals each were used at each dose level indicated in tables 3 and 4 below in the drug treated groups. In both Examples 7 and 8, for both the control group and the drug treated groups, tumors were induced by IP inoculation of tumor seed cells on day zero followed by initiation of drug treatment on day one. For both Examples 7a and 8, saline with tween/80 was used as the drug vehicle. For Examples 7b and 7c, water was used as the drug vehicle.

In both the control group and the drug treated group in Examples 7 and 8, the test animals were inoculated on day zero IP with $10^6$ seed cells of P388 lymphocytic leukemia. In both Examples 7 and 8, treatment of the drug group was started on day one and the drug was given IP once daily for nine days. Day six was utilized as the cut-off date for death attributable to toxicity of the drug. In only one instance, in Example 7b, was animal mortality attributed to drug toxicity. Efficacy of treatment was determined by comparing median survival time of the drug treated animals compared to median survival time of the control animals, and is expressed as percentage increase of treated animals/control animals (T/C) as per Example 5.

Example 7a

In this example drug treated animals were injected IP with the dose level noted in Table 3 below. Six animals were treated at each dose level. No control animals survived beyond day 18 with a median death date of day 12.6. The median death day of the drug treated animals is as shown in Table 3a below. At the 50 mg/Kg level, one drug treated animal survived and was judged as a no-take.

Example 7b

This example was performed as per Example 7a at dose levels as noted in Table 3b below. A survivor at both the 700 and 800 mg/Kg level was judged as cured. No controls survived beyond day 12 and the mean death day of the control group was day 11.

Example 7c

This example was run as per Example 7a above at dose levels noted in Table 3c below. All controls were dead by day 14 with a mean death date of day 11.9. At the 500 mg/Kg level, one animal was judged as a cure.

Example 8

Compound 2, 2-(2,3,5-tri-O-acetyl-β-D-ribofuranosyl)thiazole-4-carboxamide was tested as per Example 7a above at dose levels noted in Table 4 below. No controls survived beyond day 18 with an average death date of day 12.6. At the 50 mg/Kg level, one surviving animal was judged as a no-take.

Both compounds 1 and 2 are indicated as being active antitumor agents in the multiple dose studies noted in Examples 7 and 8.

TABLE 3a

| Drug Dose mg/Kg | Treated Group Survival Time | Control Group Survival Time | Percent Treated Animals/ Control Animals |
|---|---|---|---|
| 400 | 20.3 | 12.6 | 161% |
| 200 | 19.0 | | 150% |
| 100 | 18.3 | | 145% |
| 50 | 15.3 | | 121% |
| 25 | 14.3 | | 113% |
| 12.5 | 13.9 | | 110% |

TABLE 3b

| Drug Dose mg/Kg | Treated Group Survival Time | Control Group Survival Time | Percent Treated Animals/ Control Animals |
|---|---|---|---|
| 800 | 27.0 | 11.0 | 245% |
| 700 | 27.0 | | 245% |
| 600 | 25.8 | | 234% |
| 500 | 21.8 | | 190% |
| 400 | 24.7 | | 224% |
| 300 | 21.8 | | 198% |

TABLE 3c

| Drug Dose mg/Kg | Treated Group Survival Time | Control Group Survival Time | Percent Treated Animals/ Control Animals |
|---|---|---|---|
| 800 | 11.8 | 11.9 | 99% |
| 700 | 10. | | 86% |
| 600 | 28.3 | | 237% |
| 500 | 25.0 | | 210% |
| 400 | 24.0 | | 201% |
| 300 | 23.0 | | 193% |

TABLE 4

| Drug Dose mg/Kg | Treated Group Survival Time | Control Group Survival Time | Percent Treated Animals/ Control Animals |
|---|---|---|---|
| 400 | 20.3 | 12.6 | 161% |
| 200 | 19.0 | | 150% |
| 100 | 18.3 | | 145% |
| 50 | 15.3 | | 121% |
| 25 | 14.3 | | 113% |
| 12.5 | 13.7 | | 110% |

Compound 1 is indicated as being active against L—1210 lymphoid leukemia as per Example 9, and successfully passed the DN 2 criteria of the National Cancer Institute testing. For Example 9a and 9b, $CD_2F_1$ male mice were used and challenged with L—1210 lymphoid leukemia. The mean survival time of the test animals was compared to appropriate control animals and based on this criteria, compound 1 was considered as an active antitumor agent. The test period was thirty days. Tests results are expressed at T/C as per Example 5.

In Example 9a, 24 control animals were used and six test animals at each drug dose level as is indicated below in table 9a. In Example 9b, forty control animals were used and ten test animals each at drug dose levels as shown in table 9b below. For both the control group and the drug test group, tumors were induced by IP inoculation of tumor seed cells on day zero followed by initiation of drug treatment on day one. For Example 9a, water was used as the drug vehicle and for Example 9b saline was used as the drug vehicle.

In both the control groups and the drug treated groups in Examples 9a and 9b, the test animals were inoculated on day zero IP with $10^6$ seed cells of L—1210 lymphoid leukemia. For Example 9a, drug treatment was started on day one and compound 1 given once daily for nine days. Day five was utilized as the cut-off date for death attributable to toxicity of the drug. In only one instance in Example 9b was mortality attributed to drug toxicity. Efficacy of treatment was determined by comparing mean survival time of drug treated animals with mean survival time of the control animals and is expressed as percentage increase of treated animals/control animals (T/C) as per Example 5.

Example 9a

In this example, the drug treated animals were injected IP with dose levels noted in table 5a below. Six animals were treated at each dose level. No control animals survived beyond day ten with a mean death date of day 9.7. The mean death day of the drug treated animals is as shown in table 5a below.

Example 9b

In this example, drug treated animals were injected IP with the dose level noted in table 5b below. Ten animals were treated at each dose level. No control animals survived beyond day ten with a mean death date of day 9.0. The mean death day of the treated animals is as shown in table 5b below.

TABLE 5a

| Drug Dose mg/Kg | Treated Group Survival Time | Control Group Survival Time | Percent Treated Animals/ Control Animals |
|---|---|---|---|
| 400 | 18.7 | 9.7 | 192% |
| 200 | 15.3 | | 157% |
| 100 | 14.0 | | 144% |
| 50 | 13.2 | | 136% |
| 25 | 12.8 | | 131% |

TABLE 5b

| Drug Dose mg/Kg | Treated Group Survival Time | Control Group Survival Time | Percent Treated Animals/ Control Animals |
|---|---|---|---|
| 800 | 15.4 | 9.0 | 171% |
| 600 | 20.7 | | 230% |
| 400 | 20.2 | | 224% |
| 200 | 16.4 | | 182% |
| 100 | 16.5 | | 183% |

Compound 1 is indicated as being active against Lewis lung carcinoma as per Example 10 and successfully passed the DN 2 criteria of the National Cancer Institute Testing. For Example 10, $B_6D_2F_1$ male mice were used and challenged with Lewis lung carcinoma. The median survival time of the test animals was compared to appropriate control animals and based on this criteria compound 1 was considered as an effective antitumor agent.

In Example 10, forty control animals were used and ten test animals each at dose levels indicated below in Table 6. For both the control group and the drug treated group, tumors were induced by IV injection on day zero followed by initiating of drug treatment on day one. For Example 10 water was used as the drug vehicle.

In both the control group and the drug treated group in Example 10, the animals were inoculated on day zero with a homogenate of $10^6$ seed cells of Lewis lung carcinoma. For Example 10, drug treatment was started on day one and compound 1 given once daily for nine days. Day five was utilized as the cut-off day for deaths attributable to toxicity of the drug. There was no mortality attributable to drug toxicity in this example. Efficacy of treatment was determined by comparing median survival time of drug treated animals with median survival time of the control animals and is expressed as percentage increase of treated animals/control animals (T/C) as per Example 5.

The test period was for sixty days and at the end of the sixty day period all animals surviving in the test group were evaluated as either cured, no-takes, or tumor survivors as per Example 5 above.

Example 10

In this example, the drug treated animals were injected IP with the dose level noted in table 6 below. Ten animals were treated at each dose level. No control animals survived beyond day 23 with a median death day of day 18.4. At the test levels of 400, 200 and 25 mg/Kg all test animals survived the sixty day test period. Because of this fact, the T/C ratio noted in table 6 below is a constant figure based on assigning survival day rate of sixty to the treated animals and a median death date of 18.4 days to the control animals.

In Example 10 at both the 200 and 400 mg/Kg level, all ten surviving test animals were judged as cured. At the 100 mg/Kg level, there were eight cures and one tumor survivor with one death noted on day 46. At the 50 mg/Kg level, there were nine cures and one death on day 47.

Compound 1 is indicated as being an active antitumor agent in the multiple dose studies noted in Example 10.

TABLE 6

| Drug Dose mg/Kg | Treated Group Survival Time | Control Group Survival Time | Percent Treated Animals/ Control Animals |
|---|---|---|---|
| 400 | 60 | 18.4 | 326% |
| 200 | 60 | | 326% |
| 100 | 60 | | 326% |
| 50 | 60 | | 326% |
| 25 | 60 | | 326% |

As is shown in Example 10 above, compound 1 shows outstanding activity against Lewis lung carcinoma. Lewis lung carcinoma is an excellent example of a metastatic tumor system. The tests and control animals of Example 10 were inoculated IV with a homogenate of the tumor. Dramatic expression of this tumor is then seen in the lungs. As was noted previously, the ability to metastasize is a property that uniquely characterizes a malignant tumor from a benign tumor. In Example 10, not only was the median survival time of drug treated animals dramatically extended but, at the cessation of the test period, except at one dose level, at least 80% cures were noted and at two of those levels 100% cures were present.

Example 11

Compound 3, 2-(5-O-phosphoryl-β-D-ribofuranosyl)thiazole-4-carboxamide is indicated as being active against L—1210 Lymphoid leukemia as per example 11a and 11b. These examples were performed essentially as per example 9 above except as noted. The compound dosage test dosage levels are as noted in tables 7a and 7b below for Example 11a and 11b respectively. In these examples, thirty-six coated animals were used and six test animals each at the drug dose levels as is shown in tables 7a and 7b. Saline was used as the drug vehicle. No drug toxicity was noted in the test animals for either examples 11a or 11b. No control animals survived beyond day 10 in example 11a with a mean death date of day 8.3 and beyond day eleven in example 11b with a mean death date of day 10.1.

For both the control groups and the drug test groups, tumors were induced by IP inoculation of tumor seed cells on day zero followed by initiation of drug treatment on day one wherein compound 3 was given once daily for 5 days. Test results are expressed as T/C as per example 5.

TABLE 7a

| Drug Dose mg/Kg | Treated Group Survival Time | Control Group Survival Time | Percent Treated Animals/ Control Animals |
|---|---|---|---|
| 800 | 16.3 | 8.3 | 196% |
| 400 | 15.2 | | 183% |
| 200 | 21.5 | | 259% |
| 100 | 15.5 | | 186% |
| 50 | 11.3 | | 136% |

TABLE 7b

| Drug Dose mg/Kg | Treated Group Survival Time | Control Group Survival Time | Percent Treated Animals/ Control Animals |
|---|---|---|---|
| 600 | 16.2 | 10.1 | 160% |
| 400 | 15.7 | | 155% |
| 200 | 14.7 | | 145% |
| 100 | 12.3 | | 121% |
| 50 | 13.0 | | 128% |

Example 12

For Example 12, compound 1 was given IP to a group of $AKD_2F_1$ mice which were inflicted by intercranial inoculation with Lewis lung seed cells to establish brain tumors. The results in Table 8 below indicate that the IP inoculation of the afflicted animals with compound 1 resulted in reduction of the brain tumors indicating successful crossing of the blood brain barrier by compound 1 following the IP injection into the afflicted animals.

For this test, 32 control animals were used and no control animals survived beyond day eleven with a mean death date of day 9.6 for the controls. Eight test animals were used for each drug dose level with the exception of the 300 mg/Kg level as is shown in table 8 below. Water was used as the drug vehicle. For both the control group and the test group, tumors were induced on day zero with initiation of drug treatment on day one where compound 1 was given once daily for nine days. Test results are expressed as T/C as per example 5.

TABLE 8

| Drug Dose mg/Kg | Treated Group Survival Time | Control Group Survival Time | Percent Treated Animals/ Control Animals |
|---|---|---|---|
| 800 | 21.3 | 9.6 | 221% |
| 700 | 20.3 | | 211% |
| 600 | 20.3 | | 211% |
| 300 | 20.5 | | 213% |
| 150 | 20.5 | | 197% |
| 75 | 19.0 | | 185% |
| 37.5 | 16.0 | | 166% |
| 25.0 | 16.6 | | 172% |

In a significant number of disease states of the brain of both pathogenic and host dysfunction origins, treatment is inhibited by the lack of drug transfer across the blood brain barrier. In certain instances wherein appropriate treatment of a disease state is known, complications can arise in treating these diseases when they are located intercranially because of the lack of transfer across the blood brain barrier of effective concentrations of appropriate chemotherapeutic agents. The indication, as seen in table 8, that compound 1 successfully crosses the blood brain barrier is thus very promising for the treatment of brain tumors.

The following representative examples, 13 through 17, are given for the formulations of the active compound of the invention in illustrative pharmaceutical compositions utilizing illustrative carriers. In these examples, example 13 illustrates the use of the compounds of the invention in injectables suitable for intravenous or other types of injection into the host animal. Example 14 is directed to an oral syrup preparation, Examples 15 to an oral capsule preparation and Example 16 to oral tablets. Example 17 is

directed to use of the compounds of the invention in suitable suppositories. For example 13 through 17 the ingredients are listed followed by the methods of preparing the composition.

Example 13
Injectables

Example 13a Compound 1

| | |
|---|---|
| Compound 1 | 250 mg — 1000 mg |
| Water for Injection USP q.s. | |

Compound 1 is dissolved in the water and passed through a 0.22 μ filter. The filtered solution is added to ampoules or vials, sealed and sterilized.

Example 13b Compound 3

| | |
|---|---|
| Compound 3 as a Sodium Salt | 250 mg — 1000 mg |
| Water for Injection USP q.s. | |

Prepared as per Example 3a, above.

Example 14
Syrup
Example 14a Compound 1
250 mg Active ingredient/5 ml syrup

| | |
|---|---|
| Compound 1 | 50 g |
| Purified Water USP | 200 ml |
| Cherry Syrup q.s. or | 1000 ml |

Compound 1 is dissolved in the water and to this solution the syrup is added with mild stirring.

Example 14b Compound 3
250 mg Active Ingredient/5 mls syrup

| | |
|---|---|
| Compound 3 as a Sodium Salt | 50.0 g |
| Purified Water USP q.s. or | 200 ml |
| Cherry Syrup q.s. ad | 1000 ml |

Prepared as per Example 14a above.

Example 15
Capsules
Example 15a Compound 1
100 mg, 250 mg or 500 mg

| | |
|---|---|
| Compound 1 | 500 g |
| Lactose USP, Anhydrous q.s. or | 200 g |
| Sterotex Powder HM | 5 g |

Combine compound 1 and the Lactose in a twin-shell blender equipped with an intensifier bar. Tumble blend for two minutes, followed by blending for one minute with the intensifier bar and then tumble blend again for one minute. A portion of the blend is then mixed with the Sterotex Powder, passed through a #30 screen and added back to the remainder of the blend. The mixed ingredients are then blended for one minute, blended with the intensifier bar for thirty seconds and tumble blended for an additional minute. Appropriate sized capsules are filled with 141 mg, 352.5 mg or 705 mg of the blend, respectively, for the 100 mg, 250 mg and 500 mg containing capsules.

Example 15b
Example 15b Compound 2
100 mg, 250 mg or 500 mg

| | |
|---|---|
| Compound 2 | 500 g |
| Lactose USP, Anhydrous q.s. or | 200 g |
| Sterotex Powder HM | 5 g |

Mix and fill as per Example 15a.

Example 15c Compound 4
100 mg, 250 mg or 500 mg

| | |
|---|---|
| Compound 4 | 500 g |
| Lactose USP, Anhydrous q.s. or | 200 g |
| Sterotex Powder HM | 5 g |

Mix and fill as per Example 15a.

Example 16
Tablets
Example 16a Compound 1
100 mg, 200 mg or 500 mg

| | |
|---|---|
| Compound 1 | 500 g |
| Corn Starch NF | 200.0 g |
| Cellulose, Microcrystalline | 46.0 g |
| Sterotex Powder HM | 4.0 g |
| Purified Water q.s. or | 300.0 ml |

Combine the corn starch, the cellulose and Compound 1 together in a planetary mixer and mix for two minutes. Add the water to this combination and mix for one minute. The resulting mix is spread on trays and dried in a hot air oven at 50°C until a moisture level of 1 to 2 percent is obtained. The dried mix is then milled with a Fitzmill through a #RH2B screen at medium speed. The Sterotex Powder is added to a portion of the mix and passed through a #30 screen, and added back to the milled mixture and the total blended for five minutes by drum rolling. Compressed tablets of 150 mg, 375 mg and 750 mg respectively, of the total mix are formed with appropriate sized punches for the 100 mg, 250 mg or 500 mg containing tablets.

Example 17
Suppositories
Example 17a Compound 1
250 mg, 500 mg or 1000 mg per 3 g

| | | | |
|---|---|---|---|
| Compound 1 | 250 mg | 500 mg | 1000 mg |
| Polyethylene Glycol 1540 | 1925 mg | 1750 mg | 1400 mg |
| Polyethylene Glycol 8000 | 825 mg | 750 mg | 600 mg |

Melt the Polyethylene Glycol 1540 and the Polyethylene Glycol 8000 together at 60°C and dissolve Compound 1 into the melt. Mold this total at 25°C into appropriate suppositories.

Example 17b Compound 2
250, 500, 1000 mg per 3 g

| | | | |
|---|---|---|---|
| Compound 2 | 250 mg | 500 mg | 1000 mg |
| Polyethylene Glycol 1540 | 1925 mg | 1750 mg | 1400 mg |
| Polyethylene Glycol 8000 | 825 mg | 750 mg | 600 mg |

Prepare as per Example 17a above.

**Claims**

1. The use of a compound having the following general formula (I)

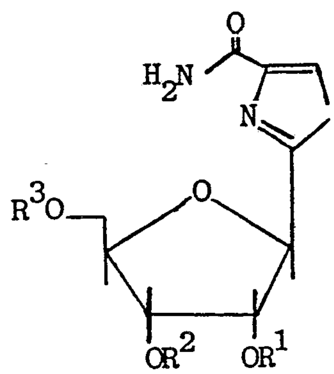

I

wherein each of $R^1$ and $R^2$, which are the same or different, is a hydrogen atom or a $C_{1-18}$ carboxylic acyl radical, and $R^3$ is a hydrogen atom, a $C_{1-18}$ carboxylic acyl radical or

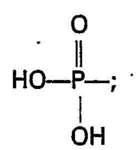

or a physiologically acceptable salt thereof; for the manufacture of a medicament for inhibiting a malignant tumor in a warm blooded animal.

2. The use of a compound of formula (I), according to claim 1, wherein each of $R^1$ and $R^2$, which are the same or different, is a hydrogen atom or a $C_{1-8}$ carboxylic acyl radical, and $R^3$ is a hydrogen atom, a $C_{1-8}$ carboxylic acyl radical or

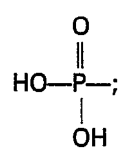

or a physiologically acceptable salt thereof.

3. The use of a compound of formula (I), according to claim 1, wherein the compound is 2-(5-O-phosphoryl-β-D-ribofuranosyl)thiazole-4-carboxamide or a physiologically acceptable salt thereof.

4. The use of a compound of formula (I), according to claim 1, wherein the compound is one wherein at least one of the radicals $R^1$, $R^2$ and $R^3$ is a benzoate ester radical.

5. The use of a composition containing, in addition to a physiologically acceptable carrier and, as an active ingredient, an effective amount of a compound as defined in claim 1, or a physiologically acceptable salt thereof, for the manufacture of a medicament for inhibiting a malignant tumor in a warm blooded animal.

6. The use according to claim 5, wherein the active compound is 2-β-D-ribofuranosylthiazole-4-carboxamide.

7. The use according to claim 5, wherein the active compound is a carboxylic acyl ester derivative of 2-β-D-ribofuranosyl)thiazole-4-carboxamide.

8. The use according to claim 7, wherein the carboxylic acyl ester is an acetate ester.

9. The use according to claim 7, wherein the carboxylic acyl ester is a benzoate ester.

10. The use according to claim 5, wherein the active compound is a phosphoryl ester of 2-β-D-ribofuranosylthiazole-4-carboxamide.

11. The use according to any one of claims 5 to 10, wherein the active compound is present in the composition in an amount of from 10% to 90% by weight of the total composition.

12. A process for producing a compound having the following general formula:

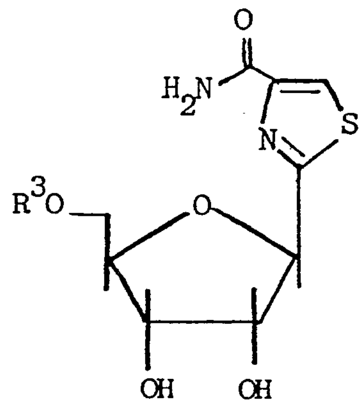

wherein $R^3$ is a $C_{1-18}$ carboxylic acyl radical or

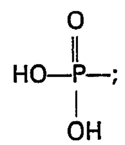

which process comprises reacting the compound 2-β-D-ribofuranosylthiazole-4-carboxamide in the presence of a base with a carboxylic acyl anhydride, a carboxylic acyl chloride, a phosphoric acid anhydride or a phosphoric acid chloride.

13. A process for producing 2-(5-O-phosphoryl-β-D-ribofuranosylthiazole-4-carboxamide, which process comprises reacting the compound 2-β-D-ribofuranosylthiazole-4-carboxamide in the presence of a base with phosphoryl chloride in a polar solvent; and treating the reaction mixture with water and isolating the desired product.

14. A process according to claim 13, wherein the desired product is isolated by desalting the reaction mixture with activated charcoal and eluting the product out of the charcoal.

**Patentansprüche**

1. Verwendung einer Verbindung mit der folgenden allgemeinen Formel (I)

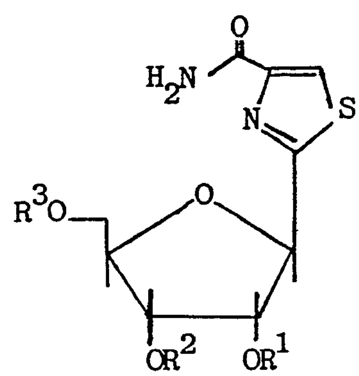

I

worin jedes von $R^1$ und $R^2$, die gleich oder verschieden sind, ein Wasserstoffatom oder ein $C_{1-18}$-carboxylischer Acylrest ist und $R^3$ ein Wasserstoffatom, ein $C_{1-18}$-carboxylischer Acylrest oder

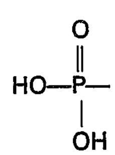

ist, oder eines physiologisch akzeptablen Salzes davon; zur Erzeugung eines Medikaments zur Hemmung eines bösartigen Tumors bei einem Warmblütler.

2. Verwendung einer Verbindung der Formel (I) nach Anspruch 1, worin jedes von $R^1$ und $R^2$, die gleich oder verschieden sind, ein Wasserstoffatom oder ein $C_{1-8}$-carboxylischer Acylrest ist und $R^3$ ein Wasserstoffatom, ein $C_{1-8}$-carboxylischer Acylrest oder

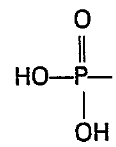

ist; oder eines physiolosich akzeptablen Salzes davon.

3. Verwendung einer Verbindung der Formel (I) nach Anspruch 1, worin die Verbindung 2-(5-O-Phosphoryl-β-D-ribofuranosyl)-thiazol-4-carboxamid oder ein physiolosich akzeptables Salz davon ist.

4. Verwendung einer Verbindung der Formel (I) nach Anspruch 1, worin die Verbindung eine solche ist, worin zumindest einer der Reste $R^1$, $R^2$ und $R^3$ ein Bezoatesterrest ist.

5. Verwendung einer Zusammensetzung, welche zusätzlich zu einem physiologisch akzeptablen Träger und als aktives Ingrediens eine wirksame Menge einer Verbindung, wie in Anspruch 1 definiert, oder ein physiologisch akzeptables Salz davon enthält, zur Erzeugung eines Medikaments zur Hemmung eines bösartigen Tumors bei einem Warmblütler.

6. Verwendung nach Anspruch 5, worin die aktive Verbindung 2-β-D-Ribofuranosylthiazol-4-carboxamid ist.

7. Verwendung nach Anspruch 5, worin die aktive Verbindung ein carboxylisches Acylesterderivat von 2-β-D-Ribofuranosylthiazol-4-carboxamid ist.

8. Verwendung nach Anspruch 7, worin der carboxylische Acylester ein Acetatester ist.

9. Verwendung nach Anspruch 7, worin der carboxylische Acylester ein Benzoatester ist.

10. Verwendung nach Anspruch 5, worin die aktive Verbindung ein Phosphorylester von 2-β-D-Ribofuranosylthiazol-4-carboxamid ist.

11. Verwendung nach irgendeinem der Ansprüche 5 bis 10, worin die aktive Verbindung in der Zusammensetzung in einer Menge von 10 bis 90 Gew.-% Gesamtzusammensetzung vorhanden ist.

12. Verfahren zur Herstellung einer Verbindung mit der folgenden allgemeinen Formel:

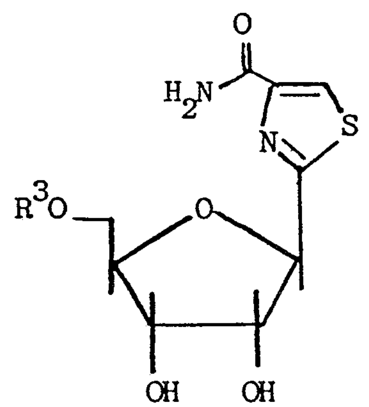

worin $R^3$ ein $C_{1-18}$-carboxylischer Acylrest oder

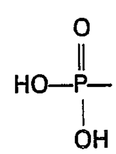

ist; welches Verfahren die Umsetzung der Verbindung 2-β-D-Ribofuranosylthiazol-4-carboxamid in Anwesenheit einer Base mit einem carboxylischen Acylanhydrid, einem carboxylischen Acylchlorid, einem Phosphorsäureanhydrid oder einem Phosphorsäurechlorid umfaßt.

13. Verfahren zur Herstellung von 2-(5-O-Phosphoryl-β-D-ribofuranosylthiazol-4-carboxamid, welches Verfahren die Umsetzung der Verbindung 2-β-D-Ribofuranosylthiazol-4-carboxamid in Anwesenheit einer Base mit Phosphorylchlorid in einem polaren Lösungsmittel; und Behandlung der Reaktionsmischung mit Wasser und Isolieren des gewünschten Produkts umfaßt.

14. Verfahren gemäch Anspruch 13, worin das gewünschte Produkt durch Entsalzen der Reaktionsmischung mit Aktivkohle und Eluieren des Produktes aus der Kohle isoliert wird.

**Revendications**

1. L'utilisation d'un composé de formule générale (I) suivante:

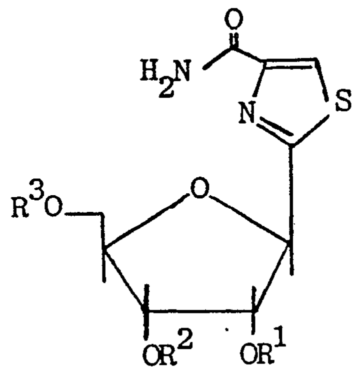

I

dans laquelle:

$R^1$ et $R^2$, qui peuvent être identiques ou différents, représentent chacun un atome d'hydrogène ou un radical acyle carboxylique en $C_1$ à $C_{18}$; et

$R^3$ représente un atome d'hydrogène, un radical acyle carboxylique en $C_1$ à $C_{18}$ ou

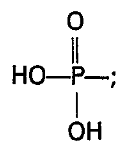

ou un de leurs sels physiologiquement acceptables; pour la préparation d'un médicament destiné au traitement d'une tumeur maligne chez un animal à sang chaud.

2. L'utilisation d'un dérivé de formule (I) selon la revendication 1, dans laquelle:

$R^1$ et $R^2$, qui sont identiques ou différents représentent chacun un atome d'hydrogène ou un radical acyle carboxylique en $C_1$ à $C_8$; et

$R^3$ représente un atome d'hydrogène, un radical acyle carboxylique en $C_1$ à $C_8$ ou

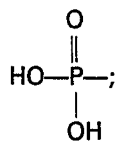

ou un de leurs sels physiologiquement acceptables.

3. L'utilisation d'un dérivé de formule (I) selon la revendication 1, dans laquelle le dérivé est le 2-(5-O-phosphoryl-β-D-ribofuranosyl)thiazole-4-carboxamide ou un de ses sels physiologiquement acceptables.

4. L'utilisation d'un dérivé de formule (I) selon la revendication 1, dans laquelle le dérivé est un produit dans lequel au moins l'un des radicaux $R^1$, $R^2$ et $R^3$ représente un radical benzoate.

5. L'utilisation d'une composition contenant, en plus d'un support physiologiquement acceptable et, à titre d'ingrédient actif, une quantité efficacé d'un dérivé selon la revendication 1 ou d'un de ses sels physiologiquement acceptables, destinée à la préparation d'un médicament destiné au traitement, d'une tumeur maligne chez un animal à sang chaud.

6. L'utilisation selon la revendication 5, dans laquelle le dérivé actif est le 2-β-D-ribofuranosylthiazole-4-carboxamide.

7. L'utilisation selon la revendication 5, dans laquelle le dérivé actif est un ester d'acyle carboxylique de 2-β-D-ribofuranosylthiazole-4-carboxamide.

8. L'utilisation selon la revendication 7, dans laquelle l'ester d'acyle carboxylique est un acétate.

9. L'utilisation selon la revendication 7, dans laquelle l'ester d'acyle carboxlique est un benzoate.

10. L'utilisation selon la revendication 5, dans laquelle le dérivé actif est un ester phosphorylé de 2-β-D-ribofuranosylthiazole-4-carboxamide.

11. L'utilisation selon l'une quelconque des revendications 5 à 10, dans laquelle le dérivé actif est présent dans la composition en quantité de 10% à 90% en poids par rapport au poids total de la composition.

12. Procédé de préparation d'un dérivé de formule générale suivante:

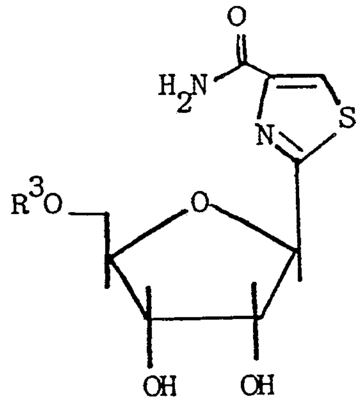

dans laquelle $R^3$ représente un radical acyle carboxylique en $C_1$ à $C_{18}$ ou

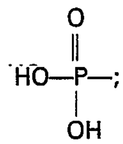

ce procédé consistant à faire réagir le composé 2-β-D-ribofuranosylthiazole-4-carboxamide en présence d'une base avec un anhydride d'acyle carboxylique, un chlorure d'acyle carboxylique, un anhydride d'acide phosphorique ou un chlorure d'acide phosphorique.

13. Un procédé de production de 2-(5-O-phosphoryl-β-D-ribofuranosyl)thiazole-4-carboxamide, ce procédé consistant à faire réagir le 2-β-D-ribofuranosylthiazole-4-carboxamide en présence d'une base avec un chlorure de phosphoryle dans un solvant polaire, puis à traiter le mélange réactionnel avec de l'eau et à isoler le produit recherché.

14. Un procédé selon la revendication 13, dans lequel le produit recherché est isolé en dessalant le mélange réactionnel par du charbon actif et en éluant le produit contenant dans la masse de charbon.